# FASCICULE DE BREVET EUROPEEN

(11) **EP 1 060 751 B1**
(45) Date de publication et mention de la délivrance du brevet: **23.08.2006**
(21) Numéro de dépôt: 00401711.7
(22) Date de dépôt: 16.06.2000
(51) Int. Cl.: A61L 9/04

(54) **Article pour la libération controlée d'une substance organique volatile**
Vorrichtung zur gesteuerten Freigabe von einem flüchtigen organischen Material
Article for the sustained release of a volatile organic substance

(30) Priorité: 18.06.1999 FR 9907761
(43) Date de publication de la demande: 20.12.2000
(73) Titulaire: Etablissements V. Mane Fils, 06620 Le-Bar-sur-Loup (FR); Dow Corning France, 69003 Lyon (FR)
(72) Inventeur: Aguadisch, Louis Michel Jacques, 06560 Valbonne (FR); Bigot, Edouard, André Henri, 06620 Le Bar-sur-Loup (FR); Colas, André Rudolf Louis, 06560 Valbonne (FR); Delpech, Guy Jean-Pierre, 06130 Grasse (FR); Fonta, Frédéric, 965 Chemin du Puy, 06600 Antibes (FR); Mane, Jean Maurice Eugène, 06130 Grasse (FR)
(74) Mandataire: Colas, Jean-Pierre

(56) Documents cités:
- WO-A-98/58624
- FR-A- 1 601 586
- FR-A- 2 689 010
- US-A- 4 703 070

## Description

L'invention concerne un article pour la libération contrôlée et sensiblement régulière d'une substance organique volatile dans l'atmosphère environnante. La substance volatile peut être notamment un parfum, ou un insecticide ou un agent repoussant les insectes.

Les élastomères de silicones sont perméables aux gaz et aux liquides. Les études de diffusion de parfum au travers d'une paroi de silicone sont nombreuses, celles à travers une matrice de silicone sont moins nombreuses. Un brevet japonais No. 82-40 558 décrit le mode de fabrication d'une matrice d'élastomère de silicone destiné à diffuser du parfum dans l'atmosphère. La composition d'élastomère de silicone est mélangée au parfum puis par ajout d'un sel organo-métallique, on déclenche la réticulation de la composition dans un moule. La matrice obtenue présente une diffusion non linéaire du parfum due à une incompatiblité du polymère et de la composition parfumante.

Le brevet américain No. 4 703 070 montre l'importance d'incorporer un agent compatibilisant pour éviter l'exsudation du parfum au cours du temps. L'invention décrit le mode de fabrication d'une matrice constituée d'un élastomère de silicone d'un agent de compatibilisation et d'un parfum. Cette matrice ne permet pas, toutefois, d'obtenir une diffusion linéaire d'une composition constante au cours du temps et pour des températures allant de 15 à 60°C.

Il existe donc un besoin pour un article permettant la libération contrôlée et sensiblement régulière d'une substance organique volatile telle qu'une composition parfumante, une composition insecticide ou une composition repoussant les insectes, sur une plage de températures d'utilisation étendue.

L'invention vise à satisfaire ce besoin.

Plus particulièrement l'invention concerne un article pour la libération contrôlée et sensiblement régulière d'une substance organique active volatile dans l'atmosphère environnante, qui comprend une matrice d'élastomère de silicone (a) de la forme voulue dans laquelle est dispersée ladite substance volatile (b) et une quantité efficace d'un agent (c) assurant la compatibilité de la substance volatile et de l'éfstomère de silicone de la matrice, l'agent compatibilisant (c) étant un solvant organique ayant un paramètre de solubilité Hildebrandt compris entre 8 et 14 (cal/cm³)^{½} et une tension de vapeur comprise entre 0,0005 et 0,8 mm de mercure (0,06 à 105 Pa)à 20°, caractérisé en ce qu'il comporte encore, dispersé dans la matrice, un agent émulsionnant (d) en une proportion efficace pour éviter un suintement ou exsudation de la substance volatile (b) à partir de la matrice (a) au cours du stockage.

Selon un mode de réalisation particulier, la substance organique volatile est un parfum ou composition parfumante.

Selon un autre mode de réalisation préféré, le constituant supplémentaire, optionnel suivant est dispersé dans la matrice :
- un fluide de silicone (e) en une proportion efficace pour faciliter la libération de la substance volatile (b) aux températures allant de 35 à 60°C. A titre indicatif, les divers constituants (a) à (e) peuvent être utilisés dans les proportions suivantes, en % en poids par rapport au poids total des constituants (a) à (e) :

| | gamme large | gamme préférée |
|---|---|---|
| - élastomère de silicone (a) : | 60-99 | 75-85 |
| - substance active volatile (b) | | |
| + solvant (c) : | 1-40 | 15-25 |
| avec la condition que le rapport en poids solvant (c)/substance (b) soit compris entre 1/99 et 99/1 | | |
| - agent émulsionnant (d) : | 0-3 | 0,5-2 |
| - fluide de silicone (e) : | 0-15 | 0-10 |

L'élastomère de silicone (a) peut être choisi parmi les divers types d'élastomère de silicone connus dans l'art. Un type préféré est celui des élastomères de silicone obtenus à partir de compositions d'élastomère de silicone qui se réticulent à la température ambiante (compositions dites "RTV") par exemple sous l'effet de l'humidité ambiante. Ces compositions d'élastomères de silicone RTV sont bien connues et disponibles dans le commerce auprès de nombreux fournisseurs. Elles sont typiquement commercialisées en deux parties, à savoir une base d'élastomère de silicone et un agent de réticulation que l'on mélange au moment de l'emploi, par exemple dans un moule. Des compositions d'élastomères se réticulant sous l'effet d'un chauffage ou d'un rayonnement conviendraient aussi, de même que des compositions réticulantes en deux, voire trois parties, réactives.

Une composition d'élastomère de silicone du commerce qui s'est avérée appropriée est constituée par la base 3481 et l'agent de réticulation DC Silastic 81 vendus par la société DOW CORNING CORPORATION.

La substance active volatile (b) peut être une substance parfumante, un insecticide ou un produit repoussant les insectes, entre autres. De préférence, la substance active volatile est une substance parfumante.

La substance parfumante peut être choisie parmi un grand nombre de composés odorants. Citons, à titre d'exemple non limitatifs, les substances appartenant aux familles suivantes :
- les hydrocarbures aromatiques, terpéniques et/ou sesquiterpéniques, notamment les huiles essentielles contenant ces molécules et plus particulièrement les huiles essentielles d'agrumes (citron, orange, pamplemousse, bergamote), noix de muscade, etc...
- les alcools aromatiques et plus particulièrement l'alcool benzylique, l'alcool phényléthylique, et l'alcool phényl propylique.
- les alcools non aromatiques primaires, secondaires ou tertiaires, saturés ou insaturés, cycliques ou acycliques et plus particulièrement le linalol, le citronnellol, le géraniol, le nérol, le dihydromyrcénol, le terpinéol, les alcools alicycliques gras dont la chaîne comporte de 4 à 10 atomes de carbone.
- les aldéhydes et plus particulièrement les aldéhydes gras saturés et insaturés alicycliques dont la chaîne carbonée comporte de 4 à 12 atomes de carbone, les aldéhydes aromatiques telles que les aldéhydes cinnamique, alpha-amyl-cinnamique et alpha hexyl-cinnamique, le lilial, et les aldéhydes aromatiques phénoliques telles que la vanilline et l'éthyl-vanilline.
- les phénols et plus particulièrement les phénols aromatiques tels que l'eugénol, l'isoeugénol ainsi que leurs éthers méthyliques.
- les esters d'acides carboxyliques, notamment les esters acétiques de l'alcool benzylique, du géraniol, du citronellol, du nérol, du terpinéol, du bornéol, et du linalol.
- les esters d'acides aromatiques tels que les benzoates et les salicylates ainsi que les cinnamates estérifiés avec des alcools de la série aliphatique possédant une chaîne de 1 à 6 atomes de carbone.
- les acides-phénol aromatiques principalement sous leur forme lactonique/aromatique tels que la coumarine et la dihydrocoumarine.
- les acides alcools carboxyliques sous leur forme lactonique et plus particulièrement les gamma octa-, gamma undéca-, gamma dodéca-lactones, les delta déca-, delta undéca-, delta dodéca-lactones sous leur forme saturée ou insaturée.
- les composés macrocycliques dont la chaîne carbonée contient de 12 à 16 atomes de carbone.
- les éthers et acétals aromatiques et/ou non aromatiques sous leur forme acyclique ou cyclique et plus particulièrement les acétals d'aldéhydes à chaîne carbonée de 4 à 10 atomes de carbone ainsi que des éthers cycliques furaniques substitués, pyraniques substitués ou non substitués.
- les composés hétérocycliques contenant un atome d'azote et plus particulièrement les dérivés indolés, ainsi que les composés hétérocycliques contenant 2 atomes d'azote et plus particulièrement ceux de la série des pyrazines.
- les cétones en particulier les cétones aromatiques telles que la 4-(p-hydroxyphényl)-2- butanone et les cétones non aromatiques saturées ou insaturées, cycliques ou non cycliques et plus particulièrement celles de la série des pyrazines.
- les sulfures, disulfures et mercaptans aromatiques ou non aromatiques.

La substance parfumante peut être constituée d'un seul composé odorant ou d'un mélange de tels composés.

Les parfums hespéridés, fleuris et fruités se sont montrés particulièrement appropriés.

Le solvant (c) est le constituant essentiel de l'article de l'invention.

Le solvant à utiliser doit avoir une tension de vapeur comprise entre 0,0005 et 0,8 mm de Hg (0,06 et 105 Pa) à 20°C, de préférence entre 0,001 et 0,6 mm de Hg (0,13 et 79 Pa) à 20°C.

En dessous de 0,0005 mm de Hg le solvant est insuffisamment volatil et a tendance à s'accumuler sur la surface extérieure de la matrice en formant un film gras et déplaisant. Au-dessus de 0,8 mm de Hg, le solvant est trop volatil et l'article résultant libère trop rapidement la substance active (b).

Le solvant (c) doit aussi avoir un paramètre de solubilité Hildebrandt δ compris entre 8 et 14 (cal/cm³)^{1/2}, de préférence entre 10 et 12 (cal/cm³)^{1/2}. afin d'assurer compatibilité satisfaisante entre l'élastomère de silicone de la matrice et la substance active (b). En dessous d'une valeur de 8 (cal/cm³)^{1/2} et au-dessus d'une valeur de 14 (cal/cm³)^{1/2}, on n'obtient pas une compatibilité convenable.

On donne ci-après, à titre illustratif et non limitatif, quelques exemples de solvants satisfaisants aux critères ci-dessus :

| | δ, (cal/cm³)^{1/2} | Tension de vapeur, mm de Hg |
|---|---|---|
| -éther monométhylique du dipropylèneglycol (1) | 10,35 | 0,0451 |
| -éther éthylique du diéthylèneglyool (2) | 10,95 | 0,0693 |
| -3-méthyl-3-méthoxy-butanol (3) | 9,33 | 0,5 |
| -éther méthylique du diéthylèneglycol | 11,40 | 0,1086 |
| -éther méthylique du triéthylèneglycol | 10,96 | 0,0037 |
| -éther n-butylique du diéthylèneglycol | 10,30 | 0,0120 |
| -éther hexylique du diéthylèneglycol. | 9,91 | 0,0018 |

(1) disponible sous la dénomination Dowanol DPM auprès de la société DOW CHEMICAL CORPORATION
(2) disponible sous la dénomination Emkanol EMK auprès de la société ICl
(3) ci-après, en abrégé, MMB

La somme du solvant (c) et de la substance active (b) doit représenter 1 à 40% de la somme des ingrédients (a) à (e), de préférence 15 à 25%. Le rapport en poids solvant/substance active volatile n'est pas très critique et peut varier de 1/99 à 99/1. De préférence ce rapport vaut de 3/7 à 2/8 en particulier environ 1/3.

L'emploi d'un fluide de silicone (e) est recommandé pour les articles dont on désire qu'ils puissent servir à des températures relativement élevées, à savoir de 35 à 60°C, car ce constituant présente habituellement une volatilité modérée aux températures ambiantes normales (20°C). L'addition de ce constituant optionnel permet d'obtenir une libération sensiblement linéaire même lorsque la température ambiante est élevée (35-60°C).

Comme fluide de silicone (e) on peut utiliser notamment les diméthicones, les cyclométhicones ou le diméthiconol. Le choix d'un fluide de silicone particulier doit se faire en fonction de l'application et de la température d'utilisation de l'article. La proportion du fluide (d) peut varier de 0 à 15% en poids. Les fluides vendus par la société Dow Corning Corporation sous les dénominations DC 345 (cyclométhicone), DC 1401 ou DC 200 se sont révélés appropriés.

L'agent émulsionnant (d) est utile pour éviter les problèmes de suintement ou d'exsudation de la substance active (b) lors du stockage de l'article. Comme agent émulsionnant (d) on peut utiliser des agents tensio-actifs de tous types tels que le Tween® 20 (polysorbate 20), l'huile de ricin hydrogénée polyéthoxylée (PE6-40), le polysorbate 40, le polysorbate 80 etc... Il suffit que l'agent (d) soit capable de maintenir à l'état d'émulsion les diverses matières premières servant à la préparation de l'article de l'invention jusqu'à la réticulation de l'élastomère de silicone.

L'article de l'invention peut être fabriqué, de façon simple, en mélangeant une base d'élastomère de silicone, la substance active volatile, le solvant et l'agent émulsionnant et, facultativement, le fluide de silicone à l'aide d'un dispositif d'agitation énergétique.

On ajoute alors l'agent de réticulation sous agitation et on place l'émulsion obtenue sous un vide partiel assurant le débullage. L'émulsion dégazée est alors injectée sous pression dans un moule dans lequel la base d'élastomère de silicone va se réticuler à froid sous l'action de l'agent de réticulation. Il ne reste plus alors qu'à démouler l'article et à le conditionner dans un emballage étanche. L'article de l'invention peut être moulé à toutes formes désirées, par exemple en forme d'une boule, d'un cube, d'un cylindre, d'une structure polygonale, etc...

L'article de l'invention, dans le mode de réalisation préféré dans lequel la substance volatile active est une substance parfumante, peut servir pour le parfumage d'un lieu, comme désodorisant, par exemple pour W-C. ou voitures, comme moyen pour parfumer du linge dans un sèche-linge, etc...

Les exemples non limitatifs suivants sont donnés dans le but d'illustrer l'invention. Le mode de préparation général sus-décrit a été utilisé dans chacun des exemples.

### Exemple 1

Un article selon l'invention pour une utilisation en sèche-linge a été préparé à partir de la formulation suivante :

| constituants | % en poids |
|---|---|
| Base d'élastomère de silicone DC 3481 | 72,5 |
| Agent de réticulation DC Silastic 81 | 7,5 |
| parfum* | 11 |
| Dowanol DPM | 4 |
| Fluide de silicone DC 345 | 4 |
| TWEEN 20 (émulsionnant) | 1 |

| | |
|---|---|
| * Dans cet exemple et les suivants, on a utilisé comme parfum de l'Alex commercialisé par les Etablissements V. MANE Fils sous le No. de catalogue M. 52548. | |

L'article obtenu, en forme de boule d'un diamètre de 35 mm, a été testé en sèche-linge avec 4 kg de linge humide à 55°C et pendant 70 mn. La perte de composés volatils (parfum + solvant) à chaque séchage est donnée par la différence de masse de l'article entre deux séchages.

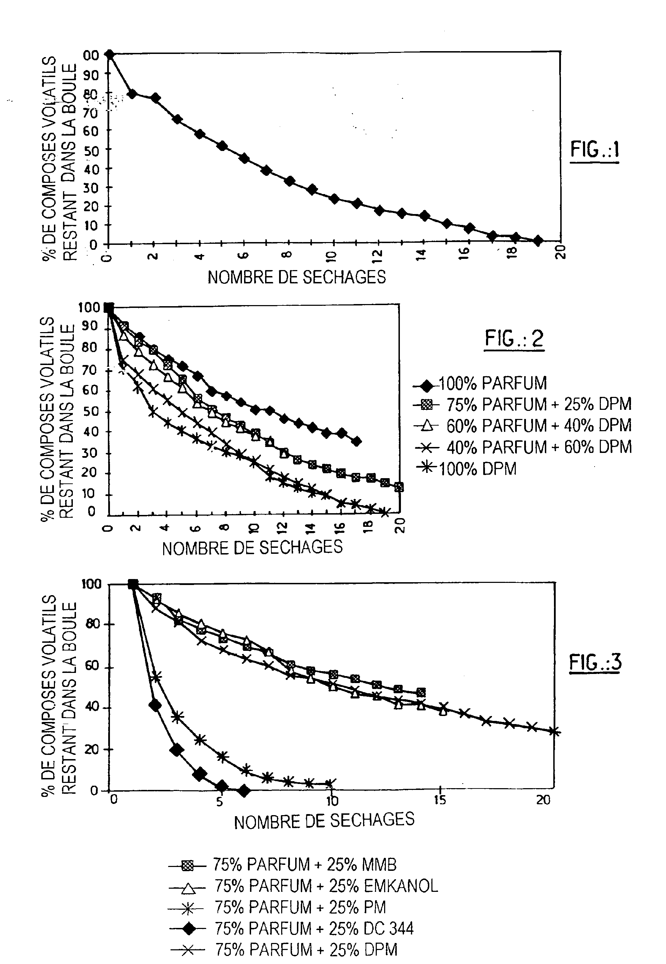

La figure 1 donne le % de composés volatils restant dans l'article en fonction du nombre de séchages. On voit clairement que la diffusion est linéaire.

### Exemple 2

### Protocole de test :

On a lavé une charge de linge de 4 kg dans une machine à laver, en procédant à un cycle d'essorage et en utilisant une lessive non parfumée.

On a ensuite mis le linge dans un sèche-linge avec une boule de silicone d'un diamètre de 35 mm et d'un poids d'environ 30 g, ayant la formulation suivante :

| Constituants | % en poids |
|---|---|
| Base d'élastomère de silicone DC 3481 | 72,50 |
| Agent de réticulation DC Silastic 81 | 7,50 |
| Parfum | 11,25 |
| Dowanol DOM | 3,75 |
| Fluide de silicone DC 345 | 4,00 |
| Tween 20 (émulsionnant) | 1,00 |

On réalise parallèlement un essai similaire avec un produit du commerce constitué d'un morceau de tissus imprégné du même parfum, qui est un produit ("drier-sheet") couramment utilisé sur le marché américain.
Cycle de séchage : 50°C pendant 70 minutes.

Le linge est ensuite évalué par un jury de 15 personnes en comparant la différence de puissance et le caractère agréable du parfumage, après le séchage (To), puis 24 heures plus tard (T₂₄).

Ce test est fait avec des boules ayant subi 2, 10 et 18 cycles de séchage en comparaison avec, à chaque fois, un "dryer-sheet" neuf.

### Résultats

### Question : Quel est le linge avant le parfum de plus puissant ?

| | Boule 2ème cyde | "Dryer sheet" | Boule 10ème cycle | "Dryer- sheet" | Boule 18ème cycle | "Dryer sheet" |
|---|---|---|---|---|---|---|
| Puissance To | 86,6% | 13,4% | 80,0% | 20,0% | 60% | 40,0% |
| Puissance T₂₄ | 93,3% | 6,7% | 80,0% | 20,0% | 80% | 20,0% |

### Question : Quel est le linge avant le parfum le plus agréable ?

| | Boule 2ème cycle | "Dryer- sheet" | Boule 10ème cycle | "Dryer- sheet" | Boule 18ème | "Dryer-sheet" |
|---|---|---|---|---|---|---|
| Puissance To | 100,0% | 0,0% | 93,3% | 6,7% | 66,6% | 33,4% |
| Puissance T₂₄ | 93,3% | 6,7% | 80,0% | 20,0% | 66,6% | 33,4% |

On peut constater que le jury, aussi bien pour la puissance que le caractère agréable du parfum, sélectionne en majorité le linge séché avec 1 boule de silicone même après 24 heures.

### Exemple 3

Cet exemple met en évidence l'importance du solvant (c). Les articles ont été préparés à partir de formulations 2 à 6 semblables à la formulation 1 de l'exemple 1, si ce n'est qu'on a fait varier les proportions relatives de solvant et de parfum tout en gardant la somme solvant + parfum égale à 15% en poids.

Le tableau ci-dessous résume les compositions des formulations 2 à 6.

| Formulation | DC 3481 | Silastic 81 | Parfum | Dowanol DPM | DC 345 | Tween 20 |
|---|---|---|---|---|---|---|
| 2 | 72,5 | 7,5 | 15 | 0 | 4 | 1 |
| 3 | 72,5 | 7,5 | 11,25 | 3,75 | 4 | 1 |
| 4 | 72,5 | 7,5 | 9 | 6 | 4 | 1 |
| 5 | 72,5 | 7,5 | 6 | 9 | 4 | 1 |
| 6 | 72,5 | 7,5 | 0 | 15 | 4 | 1 |

Les articles en forme de boule d'un diamètre de 35 mm ont été testés en sèche-linge avec 4 kgde linge humide à 60°C et pendant 70 mn.

La figure 2 montre le % de composés volatils (parfum + solvant) restant dans les articles en fonction du nombre de séchages. On voit que la libération des composés volatils est d'autant plus rapide et poussée qu'il y a davantage de solvant.

### Exemple 4

Dans cet exemple on a testé cinq solvants différents, trois conformes à l'invention et deux en dehors du cadre de l'invention, pour préparer cinq formulations 7 à 11 ayant la composition, en % en poids, indiquée dans le tableau ci-dessous.

Les deux solvants ne répondant pas aux critères de la présente invention étaient :
- l'éther méthylique du propylène-glycol (commercialisé sous la dénomination DOWANOL PM par la société DOW CHEMICAL CORPORATION), ayant un paramètre de solubilité Hildebrandt de 11,1 (callcm³)^{1/2} et une tension de vapeur de 8,74 mm de Hg à 20°C
- une cyclométhicone (commercialisée sous la dénomination DC 344 par la société DOW CORNING) ayant un paramètre de solubilité Hildebrandt de 5,98 (cal/cm³)^{½} et une tension de vapeur de 1,1649 mm de Hg à 20°C.

| | | | | | Solvant | | | |
|---|---|---|---|---|---|---|---|---|
| Formulation | DC 3481 | Silastic 81 | Parfum | MMB | Emkanol | Dowanol | Dowanol | DC344* |
| | | | | | EMK | DPM | PM** | * |
| 7 | 72,5 | 7,5 | 15 | 5 | - | - | - | - |
| 8 | 72,5 | 7,5 | 15 | - | 5 | - | - | - |
| 9 | 72,5 | 7,5 | 15 | - | - | 5 | - | - |
| 10 | 72,5 | 7,5 | 15 | - | - | - | 5 | - |
| 11 | 72,5 | 7,5 | 15 | - | - | - | - | 5 |

| | | | | | | | | |
|---|---|---|---|---|---|---|---|---|
| ** hors invention ; donné à titre comparatif. | | | | | | | | |

A partir de ces formulations, on a préparé des articles en forme de boule d'un diamètre de 35 mm et on les a testés en sèche-linge comme dans les exemples précédents.

La figure 3 montre le % de composés volatils (parfum + solvant) restant dans les articles en fonction du nombre de séchages. On voit que les trois solvants conformes à l'invention ont des comportements comparables, bien supérieurs aux deux solvants de comparaison, non conformes à l'invention.

Il va de soi que les modes de réalisation décrits ne sont que des exemples et qu'on pourrait les modifier, notamment par substitution d'équivalents techniques, sans sortir pour cela du cadre de l'invention.

## Revendications

1. Article pour la libération contrôlée et sensiblement régulière d'une substance organique active volatile dans l'atmosphère environnante, qui comprend une matrice d'élastomère de silicone (a) de la forme voulue dans laquelle est dispersée ladite substance volatile (b) et une quantité efficace d'un agent (c) assurant la compatibilité de la substance volatile et de l'élastomère de silicone de la matrice, l'agent compatibilisant (c) étant un solvant organique ayant un paramètre de solubilité Hildebrandt compris entre 8 et 14 (cal/cm³)^{1/2} et une tension de vapeur comprise entre 0,0005 of 0.8 mm de mercure (0,06 à 105 Pa) à 20°C **caractérisé en ce qu'**il comporte encore, dispersé dans la matrice, un agent émulsionnant (d) en une proportion efficace pour éviter un suintement ou exsudation de la substance volatile (b) partir de la matrice (a) au cours du stockage.

2. Article selon la revendication 1, **caractérisé en ce que** le solvant organique a une tension de vapeur comprise entre 0,001 et 0,6 mm de Hg (0,13 et 79 Pa) à 20°C.

3. Article selon la revendication 1, **caractérisé en ce que** la substance active volatile est un parfum, une composition parfumante, un insecticide ou un agent repoussant les insectes.

4. Article selon la revendication 1, **caractérisé en ce que** la substance active volatile est un parfum ou composition parfumante.

5. Article selon l'une quelconque des revendications 1 à 4, **caractérisé en ce qu'**il comporte encore
un fluide de silicone (e) en une proportion efficace pour faciliter la libération de la substance volatile (b) aux températures allant de 35 à 60°C.

6. Article selon l'une quelconque des revendications 1 à 5, **caractérisé en ce que** les constituants (a) à (e) sont utilisés dans les proportions suivantes, en % en poids par rapport au poids total des constituants (a) à (e) :
60 à 99 % d'élastomère de silicone (a)
1 à 40 % au total de substance active volatile (b) et de solvant (c)
0,5 à 2 % d'agent émulsionnant (d), et
0 à 15 % de fluide de silicone (e) ; avec la condition que le rapport en poids solvant (c)/substance (b) soit compris entre 1/99 et 99/1.

7. Article selon la revendication 6, **caractérisé en ce que** le rapport en poids solvant (c)/substance (b) est compris entre 3/7 et 2/8.

8. Article selon la revendication 7, **caractérisé en ce que** le rapport en poids solvant c)/substance (b) vaut environ 1/3.

9. Article selon l'une quelconque des revendications 1 à 8, **caractérisé en ce que** le solvant (c) présente un paramètre de solubilité Hildebrandt compris entre 10 et 12 (cal/cm³)^{1/2}.

10. Article selon la revendication 6, **caractérisé en ce que** l'élastomère de silicone (a) représente 75 à 85 %, et le total de la substance active volatile (b) et du solvant (c) représente 15 à 25 %.

11. Article selon l'une quelconque des revendications 1 à 10, **caractérisé en ce que** le solvant est de l'éther monométhylique du dipropylèneglycol

## Claims

1. An article for controlled and substantially uniform release of a volatile active organic substance into the surrounding atmosphere, said article including a silicone elastomer matrix (a) of desired shape in which are dispersed said volatile substance (b) and an effective amount of an agent (c) for compatibilizing said volatile substance and said silicone elastomer of said matrix, wherein said compatibilizer (c) is an organic solvent with a Hildebrandt solubility parameter from 8 (cal/cm³)^{1/2} to 14 (cal/cm³)^{1/2} and a vapor pressure of from 0.0005 mm to 0.8 mm of mercury (0.06 Pa to 105 Pa) at 20°C, **characterized in that** it also includes, dispersed in the matrix, an emulsifier (d) in a proportion which is effective in avoiding sweating or exudation of said volatile substance (b) from said matrix (a) during storage.

2. The article claimed in claim 1 **characterized in that** said organic solvent has a vapor pressure from 0.001 mmHg to 0.6 mmHg (0.13 Pa to 79 Pa) at 20°C.

3. The article claimed in claim 1 **characterized in that** said volatile active substance is a fragrance, a fragrancing composition, an insecticide or an insect repellent.

4. The article claimed in claim 1 **characterized in that** said volatile active substance is a fragrance or fragrancing composition.

5. The article claimed in anyone of the claims 1 to 4 **characterized in that** it also comprises:
- a silicone fluid (e) in a proportion which is effective in facilitating release of said volatile substance (b) at temperatures from 35°C and 60°C.

6. The article claimed in anyone of the claims 1 to 5 **characterized in that** the constituents (a) to (e) are used in the following proportions in percentages by weight relative to the total weight of said constituents (a) to (e) :
from 60% to 99% of said silicone elastomer (a)
from 1% to 40% in total of said volatile active substance (b) and of said solvent (c)
from 0.5% to 2% of said emulsifier (d), and
from 0% to 15% of said silicone fluid (e) ; with the condition that the weight ratio of said solvent (c) to said volatile substance (b) is from 1/99 to 99/1.

7. The article claimed, in claim 6 **characterized in that** said weight ratio of said solvent (c) to said volatile substance (b) is from 3/7 to 2/8.

8. The article claimed in claim 7 **characterized in that** said weight ratio of said solvent (c) to said volatile substance (b) is about 1/3.

9. The article claimed in anyone of the claims 1 to 8 **characterized in that** said solvent (c) has a Hildebrandt solubility parameter from 10 (cal/cm³)^{1/2} to 12 (cal/cm³)^{1/2}.

10. The article claimed in claim 6 **characterized in that** said silicone elastomer (a) represents from 75% to 85%, and the total of said volatile active substance (b) and said solvent (c) represents from 15% to 25%.

11. The article claimed in anyone of the claims 1 to 10 **characterized in that** said solvent is dipropylene glycol monomethyl ether.

## Patentansprüche

1. Vorrichtung zur gesteuerten und leicht regulierten Freisetzung eines aktiven organischen, in freier Atmosphäre flüchtigen Materials, welche eine Silikonelastomer Matrix (a) einer beliebigen Form umfasst, in welche die so genannte flüchtige Substanz (b) und eine wirksame Menge eines Stoffes (c) dispergiert werden, um die Kompatibilität der flüchtigen Substanz und des Silikonelastomers der Matrix zu gewährleisten, wobei der die Kompatibilität gewährleistende Stoff (c) ein organisches Lösungsmittel mit einem Hildebrandt Löslichkeitsfaktor zwischen 8 und 14 (cal/cm³)^{1/2} ist und einen Dampfdruck zwischen 0,0005 und 0,8 mm Quecksilber (0,06 bis 105 Pa) bei 20°C aufweist, **dadurch gekennzeichnet, dass** die Vorrichtung weiterhin ein in die Matrix dispergiertes Emulsionsmittel (d) in einem wirksamen Verhältnis umfasst, um eine Infiltration oder eine Ausschwitzung der flüchtigen Substanz (b) aus der Matrix (a) während der Lagerung zu vermeiden.

2. Vorrichtung nach Anspruch 1, **dadurch gekennzeichnet, dass** das organische Lösungsmittel eine Dampfdruck zwischen 0,001 und 0,6 mm Hg (0,13 und 79 Pa) bei 20°C aufweist.

3. Vorrichtung nach Anspruch 1, **dadurch gekennzeichnet, dass** die flüchtige Substanz ein Parfüm oder eine parfümierte Komposition, ein Insektizid oder ein Insekten abwehrendes Mittel ist.

4. Vorrichtung nach Anspruch 1, **dadurch gekennzeichnet, dass** die flüchtige Substanz ein Parfüm oder eine parfümierte Komposition ist.

5. Vorrichtung nach einem der Ansprüche 1 bis 4, **dadurch gekennzeichnet, dass** diese ein weiteres Silikonfluid (e) umfasst, in einem ausreichend wirksamen Verhältnis, um die Freisetzung der flüchtigen Substanz (b) zu unterstützen, bei Temperaturen zwischen 35 bis 60°C.

6. Vorrichtung nach einem der Ansprüche 1 bis 5, **dadurch gekennzeichnet, dass** die Grundstoffe (a) bis (e) in nachfolgend angegebenen Proportionen zur Anwendung kommen, ausgedrückt in % des Gewichts im Verhältnis zum Gesamtgewicht der Grundstoffe (a) bis (e):
- 60 bis 99% Silikonelastomer (a);
- 1 bis 40% Gesamtanteil der aktiven flüchtigen Substanz (b) und des Lösungsmittels (c);
- 0,5 bis 2% Emulsionsmittel (d), und
- 0 bis 15% Silikonfluide (e); unter der Bedingung, dass das Gewichtsverhältnis Lösungsmittel (c) : Substanz (b) innerhalb von 1/99 und 99/1 liegt.

7. Vorrichtung nach Anspruch 6, **dadurch gekennzeichnet, dass** das Gewichtsverhältnis Lösungsmittel (c) : Substanz (b) zwischen 3/7 und 2/8 liegt.

8. Vorrichtung nach Anspruch 7, **dadurch gekennzeichnet, dass** das Gewichtsverhältnis Lösungsmittel (c) : Substanz (b) in etwa bei 1/3 liegt.

9. Vorrichtung nach einem der Ansprüche 1 bis 8, **dadurch gekennzeichnet, dass** das Lösungsmittel (c) einen Hildebrandtschen Löslichkeitsparameter zwischen 10 und 12 (cal/cm³)^{1/7} aufweist.

10. Vorrichtung nach Anspruch 6, **dadurch gekennzeichnet, dass** das Silikonelastomer (a) 75 bis 85% und der Gesamtanteil der aktiven flüchtigen Substanz (b) und des Lösungsmittels (c) 15 bis 25% darstellen.

11. Vorrichtung nach einem der Ansprüche 1 bis 10, **dadurch gekennzeichnet, dass** das Lösungsmittel ein Dipropylenglykol Monomethyläther ist.
